# EUROPEAN PATENT APPLICATION

(11) **EP 2 100 574 A1**
(43) Date of publication of application: **16.09.2009**
(21) Application number: 07858241.8
(22) Date of filing: 19.11.2007
(51) Int. Cl.: A61F 11/00

(54) **AESTHETIC CORRECTOR**

(30) Priority: 21.11.2006 ES 200602517 U; 13.07.2007 ES 200701582 U
(71) Applicant: Gamero Perez, Maria Francisca, 06412 Ruecas Badajoz (ES)
(72) Inventor: SANCHEZ-MARIN PIZARRO, José María, 06071 Badajoz (ES); DIAZ PARRALEJO, Antonio, 06071 Badajoz (ES); DIAZ DIEZ, Maria Angeles, 06071 Badajoz (ES)
(74) Representative: Carpintero Lopez, Francisco
(86) International application number: PCT/ES2007/000663
(87) International publication number: WO 2008/062083

(57) **Abstract**

The invention relates to an aesthetic corrector for correcting deformations of the ear or auditory pinna, in particular excessive projection or closeness of the ears to the head, and basically made of an element (1) with two parts (2, 3), one for attaching to the user's ear and the other to the user's head, wherein said element (1) is a three-dimensional element of such thickness that when applied a set separation of the ear from the head is achieved.

## Description

### Object of the Invention

The invention relates to an aesthetic corrector of the type used to correct deformations of the ear or auditory pinna.

More particularly, the aesthetic corrector of the invention consists of an element that can correct both the excessive projection and the excessive closeness of the ears to the head, situations which are both undesirable from the aesthetic point of view.

### Background of the Invention

Today, the aesthetic appearance is a very important topic, so much so that cosmetic surgery is frequently resorted to in order to correct malformations or defects, or simply to improve one's general appearance.

One of the most common interventions today, especially among children or adolescents, is otoplasty, or ear surgery, by means of which ear defects, such as "lop ears" or excessive projection of said ears from the head, or just the opposite, i.e., excessive closeness of the ears to the head, are corrected.

To correct the first of such defects, as mentioned surgery is often resorted to. However, there are times when for economic reasons or for any other reason surgery is not possible, so other types of simpler corrective mechanisms or systems are used.

This is the case of corrective elements such as flat surfaces with one or several adhesive faces which, when located between the ear and the head, bring the ear closer to the head.

However, when the corrective element consists of a single flat surface, the corrective effect may have the opposite effect, and rather than correcting the so-called "lop ears" it may cause excessive closeness between said ears and the head.

These corrective elements further suffer from another drawback, given that since they are conceived to solve the problem of the excessive projection, they are not applicable in the opposite case, because even in the event of having several adhesive elements separated by joining elements such as strips or threads, these elements are flexible and therefore function only under traction, not under compression.

A corrective element is therefore needed which separates the ears from the head by just the right extent whether the patient's problem is the excessive projection or the excessive closeness of the ears.

In addition, in the already existing adhesive elements having two surfaces joined by one or several of the aforementioned strips, an added problem arises, which is the short duration thereof given problems relating to the adhesion of the strips or adhesive elements. Said problems can be caused either by the type of adhesive used or by the deficient distribution of stress in the surfaces having said adhesive.

More particularly, in said surfaces in which the adhesive is located, and due to the small thickness thereof, which surfaces are formed by a thin flat film, the traction exerted by the ear upon trying to become detached from the head causes in little time the corrective element to become detached, or by default, causes part of said element to become detached generally at those points corresponding to the joining points with the strips joining the surfaces, which causes the ear to maintain the correct position only at those points located on the adhesive element that have not yet become detached, to the detriment of the homogeneity of treatment, i.e., the efficacy thereof.

### Description of the Invention

This invention solves the aforementioned problems, providing a functional, efficient, comfortable and low-cost aesthetic corrector for malformations or defects of the ears, whether said defects are caused by excessive projection from or by excessive closeness to the head.

In particular, the aesthetic corrector of the invention basically consists of a flexible or elastic three-dimensional element of considerable thickness having two also three-dimensional adhesive parts, one for attaching or adhering to the ear and the other to the head, said total thickness being defined such that when applied, said ear is separated to the appropriate extent, which experts set to be approximately one centimeter.

This thickness of the corrector of the invention will therefore allow separating the ear from the head by the appropriate distance, both if the person suffers from excessive projection and from excessive closeness. This thickness can further be variable depending on each case, as can the distribution thereof, i.e., either in equal parts in both parts of the corrector or by a greater proportion in one of them, all depending on the characteristics of each case, user preferences, etc.

In addition, said thickness together with its elastic nature facilitates obtaining suitable adhesion in both parts of the corrector due to a more uniform distribution of stresses, preventing said parts from becoming partially or completely detached or lifted off from the skin, assuring correct and also homogenous fixing.

### Description of the Drawings

To complement the description being made and for the purpose of aiding to better understand the features of the invention according to a preferred practical embodiment thereof, a set of drawings is attached as an integral part of said description in which the following is depicted with an illustrative and non-limiting character:
Figure 1 shows a view of the aesthetic corrector according to a first embodiment.
Figure 2 shows a view of the aesthetic corrector according to a second embodiment.
Figure 3 shows a view of the aesthetic corrector according to a third embodiment.
Figure 4 shows a perspective view of the aesthetic corrector of the invention according to a fourth embodiment.
Figure 5 shows a perspective view of the aesthetic corrector of the invention according to a fifth embodiment, in which each part is formed by several pyramidal shapes.
Figure 6 finally shows a perspective view of the aesthetic corrector of the invention according to a sixth embodiment in which a regulating device to regulate the distance between the parts is included.

### Preferred Embodiment of the Invention

As can be seen in the figures, the aesthetic corrector of the invention consists of a flexible or elastic three-dimensional element (1) of considerable thickness having two parts (2,3), one for attaching or adhering to the ear and the other to the head.

The thickness of said parts (2,3) is such that when applied, a set separation of the ear from the head is achieved, for example, in a possible practical embodiment, by one centimeter, which is the distance that experts consider to be the most suitable.

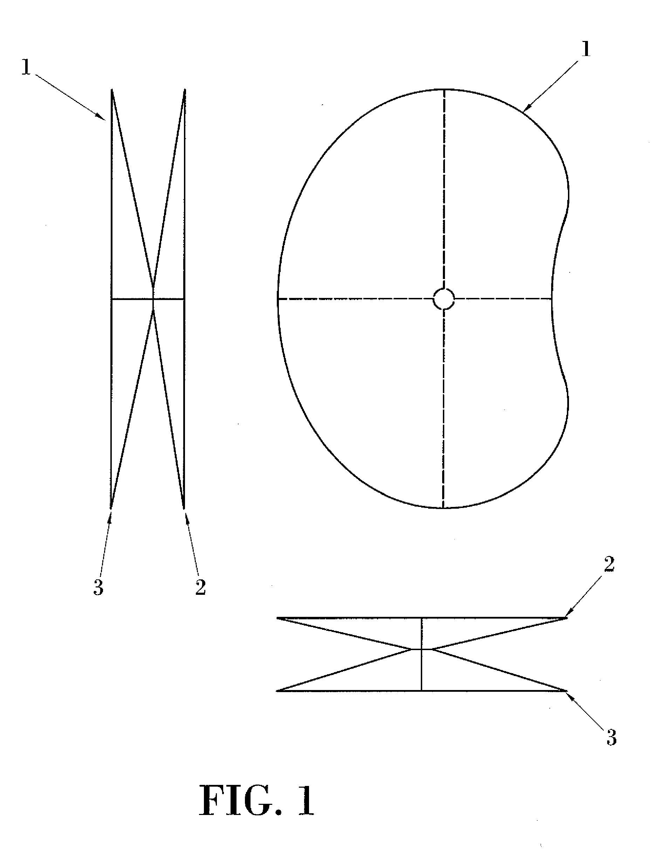

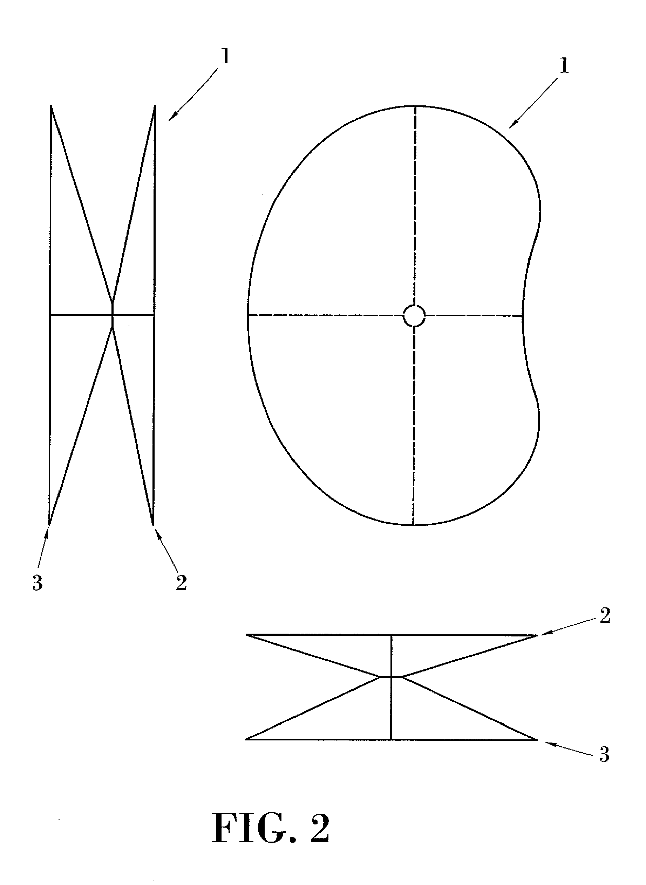

Likewise, the thickness of each of the parts (2,3) can be identical or one of them can have a larger thickness than the other one, as can be seen in Figures 1 to 3 according to a possible embodiment of the invention.

In the same manner, the thickness of each of the parts (2,3) could be uniform or not, such that the joint between said parts is located in the center thereof or, in contrast, is shifted or eccentric depending on the area of applying the corrector and for the purpose of achieving maximum adhesion and adaptability to the user's ear and to the user's head.

In the same manner, the areas of each part (2,3) for adhering to the head and the ear can have different shapes, such as circular, warped or others, depending on the area in which the corrector is going to be applied, the morphology of the user, etc.

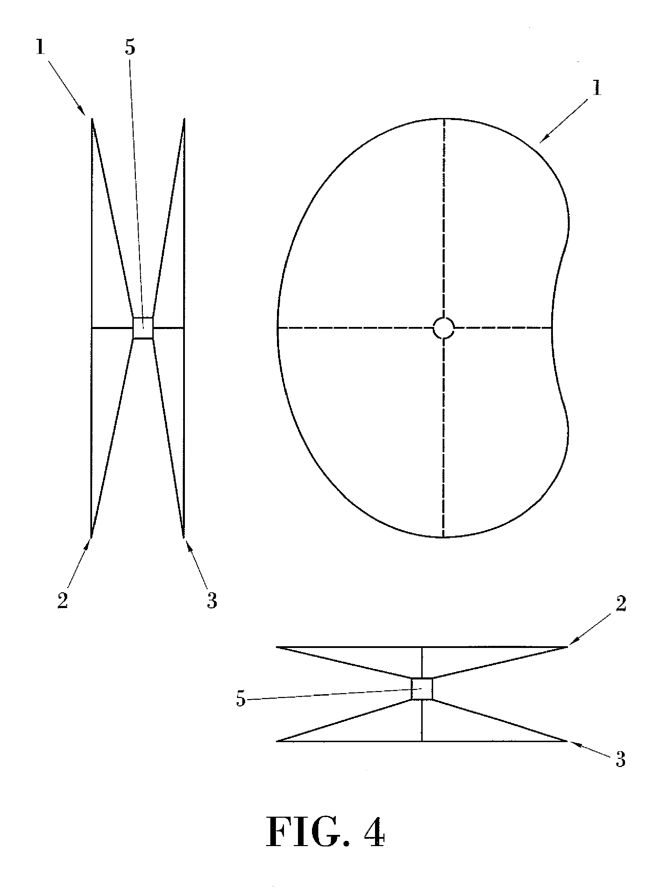

Regarding the three-dimensional shape of each of the parts (2,3), and as shown in the embodiments of Figures 1 to 4, this shape can be, for example, frusto-conical or pyramidal shape, in which their larger base is for adhering to the user and the smaller base is to be joined with the other part, such that a correct distribution of stresses is obtained while at the same time a suitable aesthetic appearance is achieved.

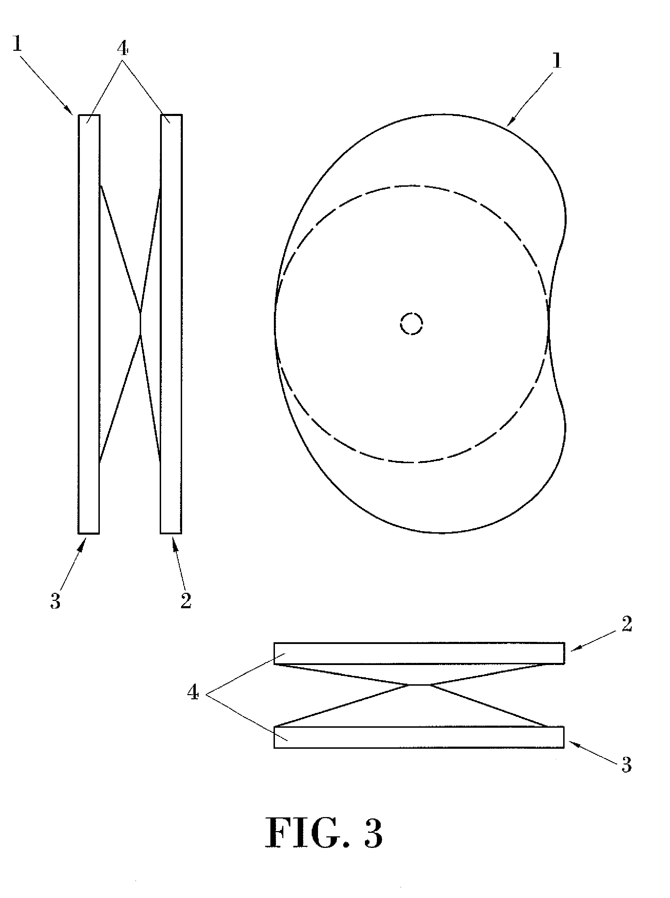

Furthermore, in another possible embodiment of the invention, and as can be seen in Figure 3, one or both of the parts (2,3) can have a base (4) of constant thickness based on which the frusto-conical or pyramidal shape is adopted for the purpose of further reinforcing adhesion to the user's skin and achieving a better and more homogenous distribution of stresses.

Alternatively, and according to another possible embodiment of the invention, the two parts (2,3) are joined to one another through an intermediate element (5).

In addition, for the purpose of achieving a perfect adhesion, each of the parts (2,3) can have, in their respective faces for attaching to the user's skin, a protective element covering it (not depicted) and which is removed in the moment in which the corrector is going to be placed in the operative position, thus preventing the loss of the adhesive (8) in handling, transport, etc.

This adhesive (8), further suited for joining the corrector to the skin of the body, can be water repellent, improving its efficacy in contact with water, hypoallergenic, biocompatible, etc.

In another possible embodiment, the faces for attaching to the user's skin may not initially incorporate any adhesive (8), and the adhesive can be applied later when it is used by the user, to that end said faces will incorporate an element or modification which facilitates gripping of the adhesive (8), such as grooves or roughnesses for example.

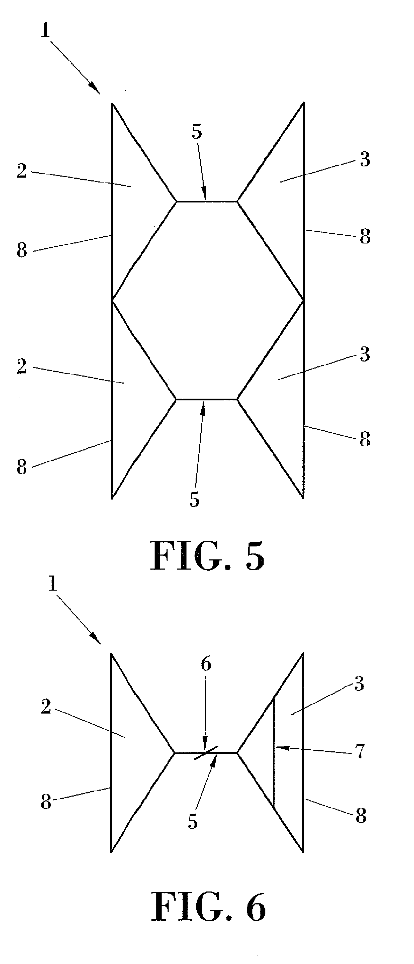

According to another possible embodiment of the invention, which can be seen in Figure 5, it shows the option of there being more than one intermediate joining element (5).

Said intermediate joining element or elements (5) according to another possible embodiment of the invention shown in Figure 5 can incorporate at least one regulating device (6), such as, for example, a threaded screw or pin, which allows the user to regulate its distance to the desired extent.

Likewise, the corrector of the invention could also in another possible embodiment incorporate a preferably internal metal or polymer frame (7), schematically depicted in Figure 6, for the purpose of serving as a support or base to allow adopting the desired shape and maintaining the corrector in an exact position, for which purpose it will have a shape and rigidity compatible with this function.

Furthermore, for the purpose of better storage and hygiene of the corrector, in another possible embodiment such corrector could be made of a transpirable material.

Finally, for the purpose of achieving a suitable aesthetic effect, the aesthetic corrector of the invention could be made in transparent materials such as silicone, for example, such that it can go unnoticed, or it can have a colored finish which covers the range of the most typical skin types, or even combining different colors and materials for each of the parts (2,3).

## Claims

1. Aesthetic corrector for correcting defects or deformations in the auditory pinna, comprising an element (1) with two parts (2,3), one for attaching to the user's ear and the other to the user's head, **characterized in that** the element (1) is a three-dimensional element of such thickness that when the corrector is applied a set separation of the ear from the head is achieved.

2. Aesthetic corrector according to claim 1, **characterized in that** the parts (2,3) are both three-dimensional with a set thickness so as to facilitate a more uniform distribution of stresses and to prevent them from becoming detached.

3. Aesthetic corrector according to claim 1, **characterized in that** the parts (2,3) have different thicknesses with respect to one another.

4. Aesthetic corrector according to any of the preceding claims, **characterized in that** the parts (2,3) have a frusto-conical or pyramidal shape, wherein their larger base is to be adhered to the user and the smaller base is to be joined with the other part, such that a correct distribution of stresses is facilitated while at the same time a suitable aesthetic appearance is achieved.

5. Aesthetic corrector according to claim 4, **characterized in that** at least one of the parts (2,3) has a base (4) of constant thickness based on which it adopts the frusto-conical or pyramidal shape.

6. Aesthetic corrector according to any of the preceding claims, **characterized in that** the two parts (2,3) are joined to one another through an intermediate element (5).

7. Aesthetic corrector according to any of claims 1 to 5, **characterized in that** the parts (2,3) are joined to one another through at least one intermediate element (5).

8. Aesthetic corrector according to any of the preceding claims, **characterized in that** at least one of the intermediate elements (5) incorporates a regulating device (6) which allows adapting the distance between the parts (2,3).

9. Aesthetic corrector according to any of the preceding claims, **characterized in that** it incorporates a metal or polymer frame (7) with a compatible shape and rigidity to maintain the ear in the desired position.

10. Aesthetic corrector according to any of the preceding claims, **characterized in that** the parts (2,3) are joined to one another eccentrically.

11. Aesthetic corrector according to any of the preceding claims, **characterized in that** each of the parts (2,3) has in the face to be attached to the user's skin a protective element covering the adhesive until the moment in which the corrector is going to be placed in operative position.

12. Aesthetic corrector according to any of claims 1 to 10, **characterized in that** each of the parts (2,3) has in the face for attaching to the skin grooves or roughnesses.

13. Aesthetic corrector according to any of the preceding claims, **characterized in that** it is made in a transparent material.

14. Aesthetic corrector according to any of the preceding claims, **characterized in that** it is made in a flexible or elastic material.

15. Aesthetic corrector according to any of the preceding claims, **characterized in that** it is made in a transpirable material.

16. Aesthetic corrector according to any of the preceding claims, **characterized in that** it is made in silicone.

17. Aesthetic corrector according to claim 1, **characterized in that** the adhesive (5) for joining each of the parts (2,3) to the user's skin is selected from hypoallergenic, water repellent or biocompatible adhesives.

18. Aesthetic corrector for correcting defects or deformations in the auditory pinna comprising an element (1) with two parts (2,3), one for attaching to the user's ear and the other to the user's head, **characterized in that** it is made in silicone.
